# EUROPEAN PATENT SPECIFICATION

(11) **EP 1 611 241 B1**
(45) Date of publication and mention of the grant of the patent: **02.11.2016**
(21) Application number: 04726021.1
(22) Date of filing: 06.04.2004
(51) Int. Cl.: C12N 15/60, C12P 13/22

(54) **METHOD FOR PRODUCING L-AMINO ACID USING BACTERIA HAVING ENHANCED EXPRESSION OF THE GENE PCKA**
VERFAHREN ZUR L-AMINOSÄUREPRODUKTION UNTER VERWENDUNG VON BAKTERIEN MIT VERSTÄRKTER EXPRESSION DES GENS PCKA
PROCEDE DE PRODUCTION DE L-ACIDES AMINES A PARTIR D'UNE BACTERIE PRESENTANT UNE EXPRESSION AMELIOREE DU GENE PCKA

(30) Priority: 07.04.2003 RU 2003109477
(43) Date of publication of application: 04.01.2006
(73) Proprietor: Ajinomoto Co., Inc., Tokyo 104-8315 (JP)
(72) Inventor: GULEVICH, Andrey, Yurievich, Moscow, 109559 (RU); BIRYUKOVA, Irina, Vladimirovna, Moscow, 115516 (RU); ZIMENKOV, Danila, Vadimovich, Moscow, 105077 (RU); SKOROKHODOVA, Aleksandra, Yurievna, Moscow, 115304 (RU); KIVERO, Aleksandr, Dmitrievich, Moscow, 115404 (RU); BELAREVA, Alla, Valentinovna, Moscow, 125252 (RU); MASHKO, Sergei, Vladimirovich, Moscow, 105043 (RU)
(74) Representative: Strehl Schübel-Hopf & Partner
(86) International application number: PCT/JP2004/004968
(87) International publication number: WO 2004/090125

(56) References cited:
- US-B1- 6 180 373
- CHAO YUN-PENG ET AL: "Enhanced conversion rate of L-phenylalanine by coupling reactions of aminotransferases and phosphoenolpyruvate carboxykinase in Escherichia coli K-12" BIOTECHNOLOGY PROGRESS, vol. 15, no. 3, May 1999 (1999-05), pages 453-458, XP002296803 ISSN: 8756-7938
- CHAO Y-P ET AL: "ALTERATION OF GROWTH YIELD BY OVEREXPRESSION OF PHOSPHOENOLPYRUVATECARBOXYLASE AND PHOSPHOENOLPYRUVATE CARBOXYKINASE IN ESCHERICHIA COLI" APPLIED AND ENVIRONMENTAL MICROBIOLOGY, WASHINGTON,DC, US, vol. 59, no. 12, December 1993 (1993-12), pages 4261-4265, XP000973822 ISSN: 0099-2240
- CHAO Y P ET AL: "Metabolic responses to substrate futile cycling in Escherichia coli." THE JOURNAL OF BIOLOGICAL CHEMISTRY. 18 FEB 1994, vol. 269, no. 7, 18 February 1994 (1994-02-18), pages 5122-5126, XP002296607 ISSN: 0021-9258
- YUN-PENG CHAO ET AL: "CONTROL OF GLUCONEOGENIC GROWTH BY PCK IN ESCHERICIA COLI" JOURNAL OF BACTERIOLOGY, WASHINGTON, DC, US, vol. 175, no. 21, 1 November 1993 (1993-11-01), pages 6939-6944, XP000610870 ISSN: 0021-9193
- MILLARD CYNTHIA SANVILLE ET AL: "Enhanced production of succinic acid by overexpression of phosphoenolpyruvate carboxylase in Escherichia coli" APPLIED AND ENVIRONMENTAL MICROBIOLOGY, vol. 62, no. 5, 1996, pages 1808-1810, XP002296608 ISSN: 0099-2240

### SEQUENCE LISTING

<110> Ajinomoto Co., Inc.
<120> METHOD FOR PRODUCING L-AMINO ACID USING BACTERIUM
   HAVING ENHANCED EXPRESSION OF pckA GENE.
<130> C128OPC4030
<140>
   <141>
<150> RU 2003109477
   <151> 2003-04-07
<160> 18
<170> PatentIn Ver. 2.1
<210> 1
   <211> 1623
   <212> DNA
   <213> Escherichia coli
<220>
   <221> CDS
   <222> (1)..(1623)
<400> 1
   atg cgc gtt aac aat ggt ttg acc ccg caa gaa ctc gag gct tat ggt    48
   Met Arg Val Asn Asn Gly Leu Thr Pro Gln Glu Leu Glu Ala Tyr Gly
    1               5                  10                  15
   atc agt gac gta cat gat atc gtt tac aac cca agc tac gac ctg ctg    96
   Ile Ser Asp Val His Asp Ile Val Tyr Asn Pro Ser Tyr Asp Leu Leu
               20                  25                  30
   tat cag gaa gag ctc gat ccg agc ctg aca ggt tat gag cgc ggg gtg   144
   Tyr Gln Glu Glu Leu Asp Pro Ser Leu Thr Gly Tyr Glu Arg Gly Val
           35                  40                  45
   tta act aat ctg ggt gcc gtt gcc gtc gat acc ggg atc ttc acc ggt   192
   Leu Thr Asn Leu Gly Ala Val Ala Val Asp Thr Gly Ile Phe Thr Gly
       50                  55                  60
   cgt tca cca aaa gat aag tat atc gtc cgt gac gat acc act cgc gat   240
   Arg Ser Pro Lys Asp Lys Tyr Ile Val Arg Asp Asp Thr Thr Arg Asp
   65                  70                  75                  80
   act ttc tgg tgg gca gac aaa ggc aaa ggt aag aac gac aac aaa cct   288
   Thr Phe Trp Trp Ala Asp Lys Gly Lys Gly Lys Asn Asp Asn Lys Pro
                   85                  90                  95
   ctc tct ccg gaa acc tgg cag cat ctg aaa ggc ctg gtg acc agg cag   336
   Leu Ser Pro Glu Thr Trp Gln His Leu Lys Gly Leu Val Thr Arg Gln
               100                 105                 110
   ctt tcc ggc aaa cgt ctg ttc gtt gtc gac gct ttc tgt ggt gcg aac   384
   Leu Ser Gly Lys Arg Leu Phe Val Val Asp Ala Phe Cys Gly Ala Asn
           115                 120                 125
   ccg gat act cgt ctt tcc gtc cgt ttc atc acc gaa gtg gcc tgg cag     432
   Pro Asp Thr Arg Leu Ser Val Arg Phe Ile Thr Glu Val Ala Trp Gln
       130                 135                 140
   gcg cat ttt gtc aaa aac atg ttt att cgc ccg agc gat gaa gaa ctg     480
   Ala His Phe Val Lys Asn Met Phe Ile Arg Pro Ser Asp Glu Glu Leu
   145                 150                 155                 160
   gca ggt ttc aaa cca gac ttt atc gtt atg aac ggc gcg aag tgc act     528
   Ala Gly Phe Lys Pro Asp Phe Ile Val Met Asn Gly Ala Lys Cys Thr
                   165                 170                 175
   aac ccg cag tgg aaa gaa cag ggt ctc aac tcc gaa aac ttc gtg gcg     576
   Asn Pro Gln Trp Lys Glu Gln Gly Leu Asn Ser Glu Asn Phe Val Ala
               180                 185                 190
   ttt aac ctg acc gag cgc atg cag ctg att ggc ggc acc tgg tac ggc     624
   Phe Asn Leu Thr Glu Arg Met Gln Leu Ile Gly Gly Thr Trp Tyr Gly
           195                 200                 205
   ggc gaa atg aag aaa ggg atg ttc tcg atg atg aac tac ctg ctg ccg     672
   Gly Glu Met Lys Lys Gly Met Phe Ser Met Met Asn Tyr Leu Leu Pro
       210                 215                 220
   ctg aaa ggt atc gct tct atg cac tgc tcc gcc aac gtt ggt gag aaa     720
   Leu Lys Gly Ile Ala Ser Met His Cys Ser Ala Asn Val Gly Glu Lys
   225                 230                 235                 240
   ggc gat gtt gcg gtg ttc ttc ggc ctt tcc ggc acc ggt aaa acc acc     768
   Gly Asp Val Ala Val Phe Phe Gly Leu Ser Gly Thr Gly Lys Thr Thr
                   245                 250                 255
   ctt tcc acc gac ccg aaa cgt cgc ctg att ggc gat gac gaa cac ggc     816
   Leu Ser Thr Asp Pro Lys Arg Arg Leu Ile Gly Asp Asp Glu His Gly
               260                 265                 270
   tgg gac gat gac ggc gtg ttt aac ttc gaa ggc ggc tgc tac gca aaa     864
   Trp Asp Asp Asp Gly Val Phe Asn Phe Glu Gly Gly Cys Tyr Ala Lys
           275                 280                 285
   act atc aag ctg tcg aaa gaa gcg gaa cct gaa atc tac aac gct atc     912
   Thr Ile Lys Leu Ser Lys Glu Ala Glu Pro Glu Ile Tyr Asn Ala Ile
       290                 295                 300
   cgt cgt gat gcg ttg ctg gaa aac gtc acc gtg cgt gaa gat ggc act     960
   Arg Arg Asp Ala Leu Leu Glu Asn Val Thr Val Arg Glu Asp Gly Thr
   305                 310                 315                 320
   atc gac ttt gat gat ggt tca aaa acc gag aac acc cgc gtt tct tat     1008
   Ile Asp Phe Asp Asp Gly Ser Lys Thr Glu Asn Thr Arg Val Ser Tyr
                   325                 330                 335
   ccg atc tat cac atc gat aac att gtt aag ccg gtt tcc aaa gcg ggc     1056
   Pro Ile Tyr His Ile Asp Asn Ile Val Lys Pro Val Ser Lys Ala Gly
               340                 345                 350
   cac gcg act aag gtt atc ttc ctg act gct gat gct ttc ggc gtg ttg     1104
   His Ala Thr Lys Val Ile Phe Leu Thr Ala Asp Ala Phe Gly Val Leu
               355                      360                      365
   ccg ccg gtt tct cgc ctg act gcc gat caa acc cag tat cac ttc ctc      1152
   Pro Pro Val Ser Arg Leu Thr Ala Asp Gln Thr Gln Tyr His Phe Leu
       370                     375                     380
   tct ggc ttc acc gcc aaa ctg gcc ggt act gag cgt ggc atc acc gaa      1200
   Ser Gly Phe Thr Ala Lys Leu Ala Gly Thr Glu Arg Gly Ile Thr Glu
   385                     390                     395                 400
   ccg acg cca acc ttc tcc gct tgc ttc ggc gcg gca ttc ctg tcg ctg      1248
   Pro Thr Pro Thr Phe Ser Ala Cys Phe Gly Ala Ala Phe Leu Ser Leu
                   405                     410                     415
   cac ccg act cag tac gca gaa gtg ctg gtg aaa cgt atg cag gcg gcg      1296
   His Pro Thr Gln Tyr Ala Glu Val Leu Val Lys Arg Met Gln Ala Ala
               420                     425                     430
   ggc gcg cag gct tat ctg gtt aac act ggc tgg aac ggc act ggc aaa      1344
   Gly Ala Gln Ala Tyr Leu Val Asn Thr Gly Trp Asn Gly Thr Gly Lys
           435                     440                     445
   cgt atc tcg att aaa gat acc cgc gcc att atc gac gcc atc ctc aac      1392
   Arg Ile Ser Ile Lys Asp Thr Arg Ala Ile Ile Asp Ala Ile Leu Asn
       450                     455                     460
   ggt tcg ctg gat aat gca gaa acc ttc act ctg ccg atg ttt aac ctg      1440
   Gly Ser Leu Asp Asn Ala Glu Thr Phe Thr Leu Pro Met Phe Asn Leu
   465                     470                     475                 480
   gcg atc cca acc gaa ctg ccg ggc gta gac acg aag att ctc gat ccg      1488
   Ala Ile Pro Thr Glu Leu Pro Gly Val Asp Thr Lys Ile Leu Asp Pro
                   485                     490                     495
   cgt aac acc tac gct tct ccg gaa cag tgg cag gaa aaa gcc gaa acc      1536
   Arg Asn Thr Tyr Ala Ser Pro Glu Gln Trp Gln Glu Lys Ala Glu Thr
               500                     505                     510
   ctg gcg aaa ctg ttt atc gac aac ttc gat aaa tac acc gac acc cct      1584
   Leu Ala Lys Leu Phe Ile Asp Asn Phe Asp Lys Tyr Thr Asp Thr Pro
           515                     520                     525
   gcg ggt gcc gcg ctg gta gcg gct ggt ccg aaa ctg taa                  1623
   Ala Gly Ala Ala Leu Val Ala Ala Gly Pro Lys Leu
       530                     535                     540
<210> 2
   <211> 540
   <212> PRT
   <213> Escherichia coli
<400> 2
   Met Arg Val Asn Asn Gly Leu Thr Pro Gln Glu Leu Glu Ala Tyr Gly
    1               5                  10                  15
   Ile Ser Asp Val His Asp Ile Val Tyr Asn Pro Ser Tyr Asp Leu Leu
               20                  25                  30
   Tyr Gln Glu Glu Leu Asp Pro Ser Leu Thr Gly Tyr Glu Arg Gly Val
           35                  40                  45
   Leu Thr Asn Leu Gly Ala Val Ala Val Asp Thr Gly Ile Phe Thr Gly
           50                  55                  60
   Arg Ser Pro Lys Asp Lys Tyr Ile Val Arg Asp Asp Thr Thr Arg Asp
    65                  70                  75                  80
   Thr Phe Trp Trp Ala Asp Lys Gly Lys Gly Lys Asn Asp Asn Lys Pro
                        85                  90                  95
   Leu Ser Pro Glu Thr Trp Gln His Leu Lys Gly Leu Val Thr Arg Gln
                   100                 105                 110
   Leu Ser Gly Lys Arg Leu Phe Val Val Asp Ala Phe Cys Gly Ala Asn
               115                 120                 125
   Pro Asp Thr Arg Leu Ser Val Arg Phe Ile Thr Glu Val Ala Trp Gln
           130                 135                 140
   Ala His Phe Val Lys Asn Met Phe Ile Arg Pro Ser Asp Glu Glu Leu
   145                 150                 155                 160
   Ala Gly Phe Lys Pro Asp Phe Ile Val Met Asn Gly Ala Lys Cys Thr
                   165                 170                 175
   Asn Pro Gln Trp Lys Glu Gln Gly Leu Asn Ser Glu Asn Phe Val Ala
               180                 185                 190
   Phe Asn Leu Thr Glu Arg Met Gln Leu Ile Gly Gly Thr Trp Tyr Gly
           195                 200                 205
   Gly Glu Met Lys Lys Gly Met Phe Ser Met Met Asn Tyr Leu Leu Pro
       210                 215                 220
   Leu Lys Gly Ile Ala Ser Met His Cys Ser Ala Asn Val Gly Glu Lys
   225                 230                 235                 240
   Gly Asp Val Ala Val Phe Phe Gly Leu Ser Gly Thr Gly Lys Thr Thr
                   245                 250                 255
   Leu Ser Thr Asp Pro Lys Arg Arg Leu Ile Gly Asp Asp Glu His Gly
               260                 265                 270
   Trp Asp Asp Asp Gly Val Phe Asn Phe Glu Gly Gly Cys Tyr Ala Lys
           275                 280                 285
   Thr Ile Lys Leu Ser Lys Glu Ala Glu Pro Glu Ile Tyr Asn Ala Ile
       290                 295                 300
   Arg Arg Asp Ala Leu Leu Glu Asn Val Thr Val Arg Glu Asp Gly Thr
   305                 310                 315                 320
   Ile Asp Phe Asp Asp Gly Ser Lys Thr Glu Asn Thr Arg Val Ser Tyr
                   325                 330                 335
   Pro Ile Tyr His Ile Asp Asn Ile Val Lys Pro Val Ser Lys Ala Gly
               340                 345                 350
   His Ala Thr Lys Val Ile Phe Leu Thr Ala Asp Ala Phe Gly Val Leu
           355                 360                 365
   Pro Pro Val Ser Arg Leu Thr Ala Asp Gln Thr Gln Tyr His Phe Leu
       370                 375                 380
   Ser Gly Phe Thr Ala Lys Leu Ala Gly Thr Glu Arg Gly Ile Thr Glu
   385                 390                 395                 400
   Pro Thr Pro Thr Phe Ser Ala Cys Phe Gly Ala Ala Phe Leu Ser Leu
                   405                 410                 415
   His Pro Thr Gln Tyr Ala Glu Val Leu Val Lys Arg Met Gln Ala Ala
               420                 425                 430
   Gly Ala Gln Ala Tyr Leu Val Asn Thr Gly Trp Asn Gly Thr Gly Lys
           435                 440                 445
   Arg Ile Ser Ile Lys Asp Thr Arg Ala Ile Ile Asp Ala Ile Leu Asn
       450                 455                 460
   Gly Ser Leu Asp Asn Ala Glu Thr Phe Thr Leu Pro Met Phe Asn Leu
   465                 470                 475                 480
   Ala Ile Pro Thr Glu Leu Pro Gly Val Asp Thr Lys Ile Leu Asp Pro
                   485                 490                 495
   Arg Asn Thr Tyr Ala Ser Pro Glu Gln Trp Gln Glu Lys Ala Glu Thr
               500                 505                 510
   Leu Ala Lys Leu Phe Ile Asp Asn Phe Asp Lys Tyr Thr Asp Thr Pro
           515                 520                 525
   Ala Gly Ala Ala Leu Val Ala Ala Gly Pro Lys Leu
   530 535 540
<210> 3
   <211> 36
   <212> DNA
   <213> Artificial Sequence
<220>
   <223> Description of Artificial Sequence: primer
<400> 3
   caatctagaa agacaggatt gggtaaatgg caaagg 36
<210> 4
   <211> 34
   <212> DNA
   <213> Artificial Sequence
<220>
   <223> Description of Artificial Sequence: primer
<400> 4
   ccgttggtcg actacagcag acgggcgcga atgg 34
<210> 5
   <211> 35
   <212> DNA
   <213> Artificial Sequence
<220>
   <223> Description of Artificial Sequence: primer
<400> 5
   tacattgtcg acatgaaact ggatgaaatc gctcg 35
<210> 6
   <211> 32
   <212> DNA
   <213> Artificial Sequence
<220>
   <223> Description of Artificial Sequence: primer
<400> 6
   tttgttgtcg accttagcta cggctgagca cg 32
<210> 7
   <211> 25
   <212> DNA
   <213> Artificial Sequence
<220>
   <223> Description of Artificial Sequence: primer
<400> 7
   tgatcggatc cgaaatgaga tataa 25
<210> 8
   <211> 24
   <212> DNA
   <213> Artificial Sequence
<220>
   <223> Description of Artificial Sequence: primer
<400> 8
   ctgcggtcga cgtccattgc tttc 24
<210> 9
   <211> 29
   <212> DNA
   <213> Artificial Sequence
<220>
   <223> Description of Artificial Sequence: primer
<400> 9
   gaccatagat ctgaattcga gctcggtac 29
<210> 10
   <211> 29
   <212> DNA
   <213> Artificial Sequence
<220>
   <223> Description of Artificial Sequence: primer
<400> 10
   acggccagat ctaagcttgc atgcctgca 29
<210> 11
   <211> 34
   <212> DNA
   <213> Artificial Sequence
<220>
   <223> Description of Artificial Sequence: primer
<400> 11
   aacagtgatc atttgcctgg cggcagtagc gcgg 34
<210> 12
   <211> 41
   <212> DNA
   <213> Artificial Sequence
<220>
   <223> Description of Artificial Sequence: primer
<400> 12
   ataaaaagct tagatctcaa aaagagtttg tagaaacgca a 41
<210> 13
   <211> 39
   <212> DNA
   <213> Artificial Sequence
<220>
   <223> Description of Artificial Sequence: primer
<400> 13
   cgtttaagat cttggtttat gtggaaattg tcgaagaga 39
<210> 14
   <211> 74
   <212> DNA
   <213> Artificial Sequence
<220>
   <223> Description of Artificial Sequence: primer
<400> 14
   gagttcttgc ggggtcaaac cattgttaac gcgcattata tctccttcga gaaacacatt 60
   tctgcgcatt tatc 74
<210> 15
   <211> 34
   <212> DNA
   <213> Artificial Sequence
<220>
   <223> Description of Artificial Sequence: primer
<400> 15
   tagcgaagat ctctgatgtc cggcggtgct tttg 34
<210> 16
   <211> 57
   <212> DNA
   <213> Artificial Sequence
<220>
   <223> Description of Artificial Sequence: primer
<400> 16
   ttcagaaaat aatcaaaaaa agttagcgtg gtgaatttacg ccccgccct gccactc 57
<210> 17
   <211> 25
   <212> DNA
   <213> Artificial Sequence
<220>
   <223> Description of Artificial Sequence: primer
<400> 17
   gcacttcgcg ccgttcataa cgata 25
<210> 18
   <211> 1188
   <212> DNA
   <213> Artificial Sequence
<220>
   <223> Description of Artificial Sequence: Cm-resistance
   gene, PppsA promoter and SD sequence of gene 10
   from phage T7
<400> 18
   ttacgccccg ccctgccact catcgcagta ctgttgtaat tcattaagca ttctgccgac 60
   atggaagcca tcacagacgg catgatgaac ctgaatcgcc agcggcatca gcaccttgtc 120
   gccttgcgta taatatttgc ccatggtgaa aacgggggcg aagaagttgt ccatattggc 180
   cacgtttaaa tcaaaactgg tgaaactcac ccagggattg gctgagacga aaaacatatt 240
   ctcaataaac cctttaggga aataggccag gttttcaccg taacacgcca catcttgcga 300
   atatatgtgt agaaactgcc ggaaatcgtc gtggtattca ctccagagcg atgaaaacgt 360
   ttcagtttgc tcatggaaaa cggtgtaaca agggtgaaca ctatcccata tcaccagctc 420
   accgtctttc attgccatac ggaattccgg atgagcattc atcaggcggg caagaatgtg 480
   aataaaggcc ggataaaact tgtgcttatt tttctttacg gtctttaaaa aggccgtaat 540
   atccagctga acggtctggt tataggtaca ttgagcaact gactgaaatg cctcaaaatg 600
   ttctttacga tgccattggg atatatcaac ggtggtatat ccagtgattt ttttctccat 660
   tttagcttcc ttagctcctg aaaatctcga taactcaaaa aatacgcccg gtagtgatct 720
   tatttcatta tggtgaaagt tggaacctct tacgtgccga tcaacgtctc attttcgcca 780
   aaagttggcc cagggcttcc cggtatcaac agggacacca ggatttattt attctgcgaa 840
   gtgatcttcc gtcacaggta tttattcggc gcaaagtgcg tcgggtgatg ctgccaactt 900
   actgatttag tgtatgatgg tgtttttgag gtgctccagt ggcttctgtt tctatcagct 960
   gtccctcctg ttcagctact gacggggtgg tgcgtaacgg caaaagcacc gccggacatc 1020
   agagatcttg gtttatgtgg aaattgtcga agagagcaga tttgcgcaac gctgggatca 1080
   gtcttaaaaa gtaaaaaaat atatttgctt gaacgattca ccgttttttt catccggtta 1140
   aatatgcaaa gataaatgcg cagaaatgtg tttctcgaag gagatata              1188

## Description

### Technical field

The present invention relates to biotechnology, specifically to a method for producing L-amino acid by fermentation using a gene derived from bacterium *Escherichia coli.* The gene is useful for improving production of aromatic L-amino acids, such as L-tryptophan, L-phenylalanine and L-tyrosine.

### Background art

Conventionally, L-amino acids have been industrially produced by fermentation methods utilizing microorganism strains obtained from natural sources, or mutants thereof which have been especially modified to enhance L-amino acid production.

Many techniques have been disclosed which describe methods for enhancing L-amino acid production. For example, such techniques include transforming a microorganism with recombinant DNA (see, for example, US patent No. 4,278,765). Many of these techniques are based on increasing the activities of enzymes involved in amino acid biosynthesis, and/or desensitizing enzymes which are involved in feedback inhibition of the target L-amino acid (see, for example, Japanese Laid-open application No56-18596 (1981), WO 95/16042 or US patent Nos. 5,661,012 and 6,040,160).

Phosphoenolpyruvate (PEP) and erythrose 4-phosphate (E4p) are the essential precursors of the common biosynthetic pathway for aromatic L-amino acids. Also, phosphoenolpyruvate is a key intermediate involved in several other cellular processes. In wild-type *E. coli,* the major PEP consumer is the phosphotransferase system (PTS). Other PEP-consuming enzymes are phosphoenolpyruvate carboxylase, which is encoded *by ppc* gene, and pyruvate kinases, which are encoded by the *pykA* and *pykF* genes. The reactions which result in PEP formation in *E. coli* are catalyzed by the glycolytic enzyme enolase and gluconeogenesis enzymes phosphoenolpyruvate synthase and phosphoenolpyruvate carboxykinase (PEP carboxykinase), which are encoded by the *eno, ppsA* and *pckA* genes, respectively (Escherichia coli and Salmonella, Second Edition, Editor in Chief: F.C.Neidhardt, ASM Press, Washington D.C., 1996).

Therefore, optimization of the specific pathways of PEP and E4P biosynthesis can improve production of aromatic L-amino acids. In particular, increasing of the PEP supply is a standard method for increasing aromatic L-amino acid production. One way of increasing the PEP supply in a cell is to avoid PEP consumption in the phosphotransferase system by use of a non-PTS carbon source. Another way is to make glucose uptake and phosphorylation PEP-independent. Yet another way to increase the PEP supply is to recycle PEP from its derivatives, such as pyruvate and oxaloacetate. The inactivation of PEP carboxylase or PEP kinases to prevent the PEP utilization in glycolysis can also be utilized. Simultaneous inactivation of both *pykA* and *pykF* genes significantly increases carbon flow from glucose to L-phenylalanine (Grinter, N.J., ChemTech, 1998, 33-37 (July)). On the other hand, significantly increased formation of L-phenylalanine in the L-phenylalanine-producing *E. coli* strain containing an inactivated *ppc* gene is accompanied by increased formation of unwanted by-products, such as acetate and pyruvate (Miller J.E. et al., J. Ind. Microbiol., 1987, 2, 143-149).

Conversion of pyruvate back into PEP can be performed by the overexpression of phosphoenolpyruvate synthase, which is encoded by the *ppsA* gene. In this case, the carbon flux will be successfully directed toward DAHP (3-deoxy-D-arabino-heptulosonate 7-phosphate) production (Patnaik R. and Liao J.C., Appl. Environ. Microbiol., 1994, 60, 3903-3908, US patents 5906925, US5985617, US6489100; PCT application WO9608567A1). It was shown that increasing the phosphoenolpyruvate synthase activity in coryneform bacterium cells leads to increased production of L-amino acids, such as L-lysine, L-glutamic acid, L-threonine, L-isoleucine and L-serine (PCT application WO0056859A1). Also, it was shown that enhancing activity of phosphoenolpyruvate synthase in coryneform bacterium cells or in bacterium belonging to the genus *Escherichia* is useful for production of L-tryptophan, L-phenylalanine, L-tyrosine, L-threonine and L-isoleucine (European patent application EP0877090A1).

It is known that growth in the presence of dicarboxylic acids or acetate, formation of PEP and intermediates of the glycolytic pathway requires, in particular, decarboxylation of oxaloacetate by PEP carboxykinase (PckA), giving PEP in an ATP-dependent reaction. On the other hand, growth in the presence of glucose exhibits the highest expression for *ppc* gene and the lowest one for *pckA* gene (Teroaka H. et al., J. Biochem. 1970, 67, 567-575; Goldie H., J. Bacteriol. 1984, 59, 832-838).

Using stoichiometric pathway analysis, a novel pathway to recycle pyruvate to PEP was proposed but not experimentally proven. This hypothetical cycle was considered to consist of PEP carboxykinase (PckA) that converted oxaloacetate to PEP and the glyoxylate shunt (Liao J.C: et al., Biotechnol. Bioeng., 1996, 52, 129-140).

Earlier it was shown that attenuation of, or switching off, the *pckA* gene and/or *yjfA* and *ytfP* open reading frames in the cells of microorganisms of the *Enterobacteriaceae* family is useful for production of L-threonine (PCT application WO0229080A2) But at present there are no reports describing the fact that enhancement of PEP carboxykinase activity in the cell of L-amino acid-producing bacterium could lead to an increase in the L-amino acid production.

### Disclosure of the invention

The present invention provides the following.
[1] A method for producing an aromatic L-amino acid selected from the group consisting of L-tryptophan, L-phenylalanine and L-tyrosine by fermentation comprising cultivating an L-amino acid-producing bacterium of *Escherichia coli,* wherein the bacterium has been modified to have enhanced activity of phosphoenol pyruvate (PEP) carboxykinase, in a culture medium to cause accumulation of L-amino acid in the culture medium, and collecting the L-amino acid from the culture medium, wherein the activity of PEP carboxykinase is enhanced by modifying an expression control sequence of the PEP carboxykinase gene on the chromosome of the bacterium so that the expression of the gene is enhanced, or by increasing copy number of the gene, and wherein the bacterium is further modified to have enhanced expression of *yddG* open reading frame by increasing the copy number of the *yddG* open reading frame, wherein the *yddG* gene can be cloned using the primers depicted in SEQ ID No. 7 and No. 8.
[2] The method according to [1] above, wherein a native promoter of said PEP carboxykinase gene is replaced with more potent promoter.
[3] The method according to [2] above, wherein said more potent promoter is ppsA promoter which is activated by FruR protein.
[4] The method according to [1] above, wherein a native Shine-Dalgarno (SD) sequence of said PEP carboxykinase gene is replaced with a more efficient SD sequence.
[5] The method according to [4] above, wherein said more potent SD sequence is SD sequence of ϕ10 gene from phage T7.
[6] The method according to any one of [1] to [5] above, wherein the PEP carboxykinase gene encodes the following protein (A) or (B):
   (A) a protein, which comprises the amino acid sequence shown in SEQ ID NO: 2 in Sequence listing;
   (B) a protein which comprises the amino acid sequence shown in SEQ ID NO: 2 in Sequence listing including deletion,
   substitution, insertion or addition of one to ten amino acids, and which has an activity of PEP carboxykinase.
[7] The method according to any one of [1] to [5] above, wherein the PEP carboxykinase gene comprises the following DNA (a) or (b):
   (a) a DNA which comprises the nucleotide sequence of the nucleotides 1 to 1623 in SEQ ID NO: 1; or
   (b) a DNA which is hybridizable with the nucleotide sequence of the nucleotides 1-1623 in SEQ ID NO:1 or a probe which can be prepared from said nucleotide sequence under stringent conditions and encodes a protein having an activity of PEP carboxykinase, wherein the stringent conditions are conditions in which washing is performed at 60°C, and at a salt concentration corresponding to 1 x SSC and 0.1 % SDS.
[8] The method according to any one of [1] to [5] above, wherein the PEP carboxykinase gene encodes a protein which comprises the amino acid sequence shown in SEQ ID NO: 2 in Sequence Listing.
[9] The method according to any one of [1] to [5] above, wherein the PEP carboxykinase gene comprises the nucleotide sequence of the nucleotides 1 to 1623 in SEQ ID NO: 1.
[10] The method according to any one of [1] to [9] above, wherein the bacterium has enhanced expression of other genes for aromatic amino acid biosynthesis.

### Brief Description of Drawing

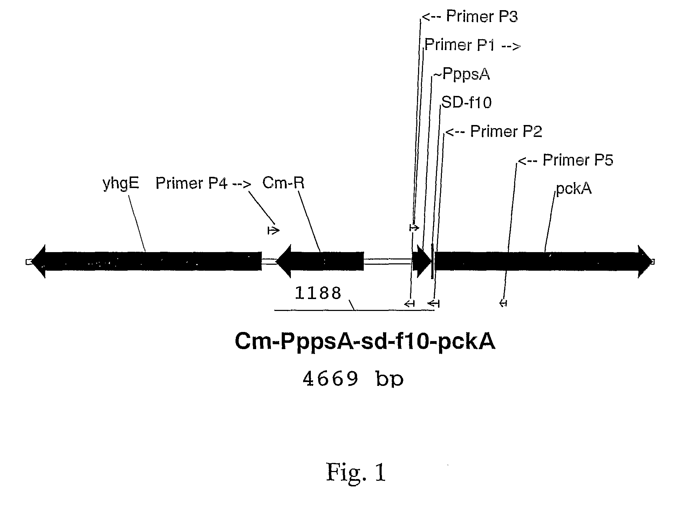

Figure 1 shows the structure of constructed chromosome region upstream of *pckA* gene.

### Best Mode for Carrying Out the Invention

The present inventors have found that enhancement of transcription and/or translation of the *pckA* gene can enhance L-tryptophan production when expressed in an L-tryptophan-producing strain. The *pckA* gene encodes PEP carboxykinase, which catalyzes conversion of oxaloacetate to PEP. Thus the present invention has been completed.

The present invention will be explained in detail below.

A bacterium used in the present invention is an L-amino acid-producing bacterium belonging to the genus *Escherichia* that has enhanced activity of a protein, and as a result, enhances production of a target L-amino acid. More specifically, the bacterium used in the present invention is an aromatic L-amino acid-producing bacterium belonging to the genus *Escherichia* that has enhanced activity of the protein of the present invention. Even more specifically, the bacterium used in the present invention is an aromatic L-amino acid-producing bacterium, such as an L-tryptophan-producing bacterium, belonging to the genus *Escherichia,* wherein the bacterium has been modified to have enhanced activity of PEP carboxykinase. More specifically, the bacterium used in the present invention harbors the DNA comprising the *pckA* gene, and has a modified expression control sequence in the chromosome of the bacterium, and has enhanced ability to produce L- tryptophan.

The term "L-amino acid-producing bacterium" means a bacterium having an ability to cause accumulation of L-amino acid in a medium when the bacterium used in the present invention is cultured in the medium. The L-amino acid producing ability may be imparted or enhanced by breeding. The term "L-amino acid-producing bacterium" used herein also means a bacterium, which is able to produce and cause accumulation of L-amino acid in a culture medium in an amount larger than a wild-type or parental strain, and preferably means that the microorganism is able to produce and cause accumulation in a medium of an amount not less than 0.5 g/L, more preferably not less than 1.0 g/L of a target L-amino acid. "L-amino acid" includes L-alanine, L-arginine, L-asparagine, L-aspartic acid, L-cysteine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine and L-valine. In the present invention, the L-amino acid L-tryptophan, L-phenylalanine and L-tyrosine are produced.

The term "a bacterium belonging to the genus *Escherichia"* means *Escherichia coli* (*E. coli*).

The term "activity of PEP carboxykinase" means an activity to catalyze the reaction of converting the oxaloacetate into phosphoenolpyruvate, while consuming ATP and releasing ADP and carbon dioxide. Activity of a PEP carboxykinase could be measured by the method described by, for example, Krebs A. and Bridger W.A. (Can. J. Biochem., 1980, 58, 309-318).

The term "modified to have enhanced activity of PEP carboxykinase" means that the activity of PEP carboxylase per cell has become higher than that of a non-modified strain, for example, a wild-type strain. For example, strains in which the number of PEP carboxykinase molecules per cell is increased, strains in which the specific activity per PEP carboxykinase molecule is increased, and so forth, are encompassed. Furthermore, a wild-type strain that serves as an object for comparison includes, for example, the *Escherichia coli* K-12. As a result of enhancement of intracellular activity of PEP carboxykinase, an effect is obtained whereby the amount of L-tryptophan which has accumulated in a medium increases.

Enhancement of PEP carboxykinase activity in a bacterial cell can be attained by enhancement of expression of a gene coding for PEP carboxykinase. As the PEP carboxykinase gene, any of the genes derived from bacteria belonging to the genus *Escherichia,* as well as genes derived from other bacteria such as coryneform bacteria, can be used. Of these, genes derived from bacteria belonging to the genus *Escherichia* are preferred.

As the gene encoding PEP carboxykinase of *Escherichia coli* (EC number 4.1.1.49), the *pckA* gene has been elucidated (nucleotide numbers 3530456 to 3532078 in the sequence of GenBank accession NC_000913.1, gi: 16131280). Therefore, the *pckA* gene can be obtained by PCR (polymerase chain reaction; refer to White, T.J. et al., Trends Genet., 5, 185 (1989)) utilizing primers prepared based on the nucleotide sequence of the gene. Genes encoding PEP carboxykinase of other microorganisms can be obtained in a similar manner.

The *pckA* gene originated from the genus of *Escherichia* or its equivalents encompasses a DNA which encodes the following protein (A) or (B):
(A) a protein which comprises the amino acid sequence shown in SEQ ID NO:2 in Sequence listing;
(B) a protein which comprises the amino acid sequence shown in SEQ ID NO:2 in Sequence listing including deletion, substitution, insertion or addition of one or several amino acids, and which has an activity of PEP carboxykinase.

The DNA coding for proteins includes a DNA coding for the protein which include deletion, substitution, insertion or addition of one or several amino acids in one or more positions on the protein (A), as long as they do not lose the activity of the protein. The number of "several" amino acids referred to herein is 2 to 10 for the protein (A). The DNA coding for substantially the same protein as the protein defined in (A) may be obtained by, for example, modification of the nucleotide sequence coding for the protein defined in (A). For example, site-directed mutagenesis can be employed so that deletion, substitution, insertion or addition of an amino acid residue or residues occurs at a specific site. Such modified DNA can be obtained by conventional methods, such as by treating with reagents or other conditions generating mutations. Such methods includes treatment of the DNA coding for proteins with hydroxylamine, or treatment of the bacterium harboring the DNA with UV irradiation or a reagent such as N-methyl-N'-nitro-N-nitrosoguanidine or nitrous acid.

The DNA coding for the proteins include variants which can be found in the different strains and variants of bacteria belonging to the genus *Escherichia* according to natural diversity. The DNA coding for such variants can be obtained by isolating the DNA, which hybridizes with *pckA* gene or a part of the gene under stringent conditions, and which encodes the protein having the activity of PEP carboxykinase. The term "stringent conditions" referred to herein include conditions under which a so-called specific hybrid is formed, and a non-specific hybrid is not formed. The stringent conditions include conditions which comprise ordinary washing conditions for Southern hybridization, i.e., 60°C, 1 x SSC, 0.1% SDS, preferably 0.1 x SSC, 0.1% SDS. As a probe for the DNA that codes for variants and hybridizes with *pckA* gene, a partial sequence of the nucleotide sequence of SEQ ID NO: 1 can also be used. Such a probe may be prepared by PCR using oligonucleotides produced based on the nucleotide sequence of SEQ ID NO: 1 as primers, and a DNA fragment containing the nucleotide sequence of SEQ ID NO: 1 as a template. When a DNA fragment of about 300 bp in length is used as the probe, the washing conditions of the hybridization can be, for example, 50°C, 2 x SSC, and 0.1% SDS.

The activity of PEP carboxykinase can be enhanced by, for example, transforming the bacterium belonging to the genus *Escherichia* with a recombinant DNA, which has been prepared by ligating a gene fragment encoding PEP carboxykinase with a vector that functions in the bacterium belonging to the genus *Escherichia,* preferably a multiple copy type vector. The activity of PEP carboxylase is enhanced as a result of the increase in copy numbers of the genes encoding the enzymes in the transformant strain.

Transformation of bacterium with a DNA coding for a protein means introduction of the DNA into bacterium cell for example by conventional methods to increase expression of the gene coding for the protein and to enhance the activity of the protein in the bacterial cell. The activity of PEP carboxykinase can also be enhanced by enhancing transcription and/or translation of a gene encoding PEP carboxykinase. Thus the bacterium used in the present invention includes one wherein the activity of the protein is enhanced by alteration of an expression control sequence of the DNA coding for protein as defined in (A) or (B) on the chromosome of the bacterium. The enhancement of gene expression can be achieved by locating the DNA under the control of a more potent promoter in place of the native promoter. The term "native promoter" means a DNA region present in a wild-type organism which is located upstream of the open reading frame (ORF) of the gene and has the function of promoting expression of the gene. Sequence of the native promoter of *pckA* gene was presented by Ramseier T.M. et al. (Mol. Microbiol., 1995, 16, 6, 1157-1169) and is available in EMBL/GenBank database under accession number U21325. Strength of a promoter is defined by frequency of RNA synthesis initiation acts. Method for evaluating the strength of a promoter is described by, for example, Deuschle U., Kammerer W., Gentz R., Bujard H. (Promoters in Escherichia coli: a hierarchy of in vivo strength indicates alternate structures. EMBO J. 1986, 5, 2987-2994).

The FruR protein (also known as Cra - Catabolite Repressor/Activator) is a global regulatory protein that modulates the direction of carbon flow. Key enzymes of most central carbohydrate metabolic pathways are the targets of a cAMP-independent mechanism of catabolite repression in enteric bacteria mediated by the FruR protein. In the presence of appropriate exogenous sugar substrates, expression of genes encoding enzymes of glycolytic and Entner-Doudoroff pathways, including some genes encoding key proteins of the PTS, is activated. At the same time, expression of genes encoding key enzymes of gluconeogenic, glyoxalate shunt, and Krebs cycle pathways is inhibited. The FruR protein regulates expression of all of these genes. The FruR represses synthesis of key enzymes of glycolytic and Entner-Doudoroff pathways and activates synthesis of key enzymes of the three other pathways (Escherichia coli and Salmonella, Second Edition, Editor in Chief: F.C. Neidhardt, ASM Press, Washington D.C., 1996). PEP carboxykinase encoded by *pckA* gene is the gluconeogenic enzyme. Since it is known that P_{ppsA} promoter is up to 20 times more activated by the FruR protein while P_{pckA} promoter is only activated by FruR up to 4 times (Saier M.H., Jr., and Ramseier T. M., J. Bacteriol., 1996, 178, 12, 3411-3417), the P_{ppsA} promoter could be assumed to be a more potent promoter then P_{pckA} promoter in the strains constitutively expressing the *fruR* gene.

The enhancement of translation can be achieved by introducing into the DNA a more efficient Shine-Dalgarno sequence (SD sequence) in the place of a native SD sequence. The SD sequence is a region upstream of the start codon of mRNA which interacts with the 16S RNA of ribosome (Shine J. and Dalgarno L., Proc. Natl. Acad. Sci. U.S.A., 1974,71,4, 1342-6). The term "native SD sequence" means SD sequence present in the wild-type organism. The nucleotide sequence of the native SD sequence of the *pckA* gene, which is a part of promoter region, was presented by Ramseier T.M. et al. (Mol. Microbiol., 1995, 16, 6, 1157-1169) and is available in EMBL/GenBank database under accession number U21325. SD sequence of the ϕ10 gene from phage T7 can be exemplified as an efficient SD sequence (Olins P.O. et al., Gene, 1988, 73, 227-235).

Methods for preparation of chromosomal DNA, hybridization, PCR, preparation of plasmid DNA, digestion and ligation of DNA, transformation, selection of an oligonucleotide as a primer, and the like may be ordinary methods well known to one skilled in the art. These methods are described in, for example, Sambrook, J., and Russell D., "Molecular Cloning A Laboratory Manual, Third Edition", Cold Spring Harbor Laboratory Press (2001) and the like.

The bacterium used in the present invention can be obtained by introduction of the aforementioned DNAs into bacterium inherently having ability to produce L-amino acid. Alternatively, the bacterium used in the present invention can be obtained by imparting to the bacterium already harboring the DNAs the ability to produce L-amino acid.

As a parent strain which is to be enhanced in activity of the protein, an L-tryptophan-producing bacterium belonging to the genus *Escherichia* such as the *E. coli* strains JP4735/pMU3028 (DSM10122) and JP6015/pMU91 (DSM10123) deficient in the tryptophanyl-tRNA synthetase encoded by mutant *trpS* gene (US patent 5,756,345); *E*. *coli* strain SV164 (pGH5) having *serA* allele free from feedback inhibition by serine (US patent 6,180,373); *E. coli* strains AGX17 (pGX44) (NRRL B-12263) and AGX6(pGX50)aroP (NRRL B-12264) deficient in the enzyme tryptophanase (US patent 4,371,614); *E. coli* strain AGX17/pGX50,pACKG4-pps in which a phosphoenolpyruvate-producing ability is enhanced (WO9708333, US patent 6,319,696) and the like may be used.

Previously, authors of the present invention identified that when the wild-type allele of the *yddG* gene, which encodes a membrane protein is not involved in biosynthetic pathway of any L-amino acid, was amplified on a multi copy vector in a microorganism, resistance to L-phenylalanine and several other amino acid analogues was imparted to the microorganism. Besides, the *yddG* gene can enhance production of L-nhenylalanine or L-tryptophan when additional copies of the gene are introduced into the cells of the respective producing strain (WO03044192). The L-tryptophan-producing bacterium can be further modified to have enhanced expression of *yddG* open reading frame or genes effective for L-tryptophan biosynthesis, which include genes of *trpEDCBA* operon, genes of common pathway for aromatic acids such as *aroF, aroG*, *aroH, aroB, aroD, aroE, aroK*, *aroL*, *aroA* and *aroC* genes, genes of L-serine biosynthesis such as *serA, serb* and *serC* genes, and the like.

The method of present invention includes a method for producing an L-amino acid comprising cultivating the bacterium used in the present invention in a culture medium, allowing the L-amino acid to be produced and accumulated in the culture medium, and collecting the L-amino acid from the culture medium. Also the method of the present invention includes a method for producing L-tryptophan comprising cultivating the bacterium used in the present invention in a culture medium, allowing L-tryptophan to be produced and accumulated in the culture medium, and collecting L-tryptophan from the culture medium.

In the present invention, cultivation, collection, and purification of L-amino acids such as L-tryptophan from the medium and the like may be performed in a manner similar to a conventional fermentation method wherein an amino acid is produced using a microorganism. A medium used for culture may be either a synthetic medium or a natural medium, so long as the medium includes a carbon source and a nitrogen source and minerals and, if necessary, appropriate amounts of nutrients which the microorganism requires for growth. The carbon source may include various carbohydrates such as glucose and sucrose, and various organic acids. Depending on the mode of assimilation of the chosen microorganism, alcohol, including ethanol, and glycerol may be used. As a nitrogen source, various ammonium salts such as ammonia and ammonium sulfate, other nitrogen compounds such as amines, a natural nitrogen source such as peptone, soybean-hydrolysate and digested fermentative microorganism can be used. As minerals, potassium monophosphate, magnesium sulfate, sodium chloride, ferrous sulfate, manganese sulfate, calcium chloride, and the like are used. Additional nutrients can be added to the medium, if necessary. For instance, if the microorganism requires tyrosine for growth (tyrosine auxotrophy) a sufficient amount of tyrosine can be added to the cultivation medium.

The cultivation is performed preferably under aerobic conditions such as by shaking and/or stirring with aeration, at a temperature between 20 to 42 °C, preferably between 37 to 40 °C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to the accumulation of the target L-amino acid in the liquid medium.

After cultivation, solids such as cells can be removed from the liquid medium by centrifugation or membrane filtration, and the target L-amino acid can be subsequently collected and purified by ion-exchange, concentration and/or crystallization methods.

### Examples

The present invention will be more concretely explained below with reference to the Examples. In the Examples, an amino acid is of L-configuration unless otherwise noted.

### Example 1: Construction of the plasmid carrying the fruR gene and the mutant serA gene encoding for a protein which is not subject to feedback inhibition by serine.

An L-tryptophan producing-strain SV164(pGH5) (US patent 6,180,373) contains the pGH5 plasmid which carries a mutant *serA5* gene coding for a protein not subject to feedback inhibition by serine. Amplification of the *serA5* gene is necessary for increasing the amount of serine, which is the precursor of L-tryptophan.

On the other hand, it is also known that P_{ppsA} promoter is activated by the FruR protein up to 20 times while P_{pckA} promoter is activated by FruR only for 4 times (Saier M.H., Jr., and Ramseier T. M., J. Bacteriol., 1996, 178, 12, 3411-3417). Since the object is to enhance activity of PEP carboxykinase by substituting the P_{pckA} promoter with the P_{ppsA} promoter, it was necessary to express the *fruR* gene constitutively.

For accomplishing the above two goals, the plasmid pGH5 in the L-tryptophan-producing strain was substituted with pMW-P_{lacUV5}-serA5-fruR plasmid, which was constructed by the following scheme.

Based on the known nucleotide sequence of pGH5 plasmid (US patent 6,180,373) the primers depicted in SEQ ID No.3 (primer A) and No. 4 (primer B) were synthesized. Primer A is complementary to a sequence at the start codon region of the *serA5* gene which has a restriction enzyme *XbaI* recognition site introduced at the 5'-end thereof. Primer B is complementary to a sequence of the termination codon region of *serA5* gene which has a restriction enzyme *SalI* recognition site at the 5'-end thereof.

The pGH5 plasmid was isolated by ordinary methods. PCR was carried out on "ThermoHybaid PCRExpress PCR system" under the following conditions: 30 sec at 95 °C, 30 sec at 55 °C, 30 sec at 72 °C, 25 cycles with a Taq polymerase (MBI Fermentas).

Concurrently, amplification of the *fruR* gene was performed. The chromosomal DNA of *E. coli* strain W 3350 was isolated by ordinary methods. Primers C (SEQ ID No. 5) and D (SEQ ID No. 6) were used. Primer C (complementary to a start codon region of *fruR* gene) contains a SalI-restriction site. Primer D (complementary to a stop codon region of *fruR* gene) contains a *Hind*III-restriction site. PCR conditions were as follows: 30 sec at 95 °C, 30 sec at 53 °C, 30 sec at 72 °C, 25 cycles with a Taq polymerase(MBI Fermentas).

The PCR fragments containing *serA5* and *fruR* genes obtained as described above were treated with *Sal*I and then ligated. The ligated product was digested by *Xba*I and *Hind*III, and inserted into the pMW-P_{lacUV5}-lacZ plasmid (Mashko S.V., et al., Biotekhnologiya (rus), 2001, 5, 3-20), which had been previously treated with the same restriction enzymes. Thus the pMW-P_{lacUV5}-serA5-fruR plasmid was obtained.

### Example 2: Cloning the yddG gene from E. coli.

The *yddG* gene, which encodes a transmembrane protein useful for L-tryptophan production (WO03/044192), was cloned using the primers depicted in SEQ ID No. 7 (primer yddg1) and No. 8 (primer yddg2). Primer yddg1 is complementary to a sequence from 91 to 114 bp downstream of the termination codon of the *yddG* gene which has a restriction enzyme *Bam*HI recognition site introduced at the 5'-end thereof. Primer yddg2 is complementary to a sequence from 224 to 200 bp upstream of the start codon of the *yddG* gene, which has a restriction enzyme *Sal*I recognition site introduced at the 5'-end thereof.

The chromosomal DNA of *E. coli* strain TG1 was prepared by ordinary methods. PCR was carried out using "Perkin Elmer GeneAmp PCR System 2400" under the following conditions: 40 sec. at 95 °C, 40 sec. at 47 °C, 40 sec. at 72 °C, 30 cycles with a *Taq* polymerase (Fermentas). The obtained PCR fragment containing *yddG* gene with its own promoter was treated with *Bam*HI and *Sal*I and inserted into the multicopy vector pAYCTER3, which had been previously treated with the same enzymes. Thus, the plasmid pYDDG2 was obtained.

The pAYCTER3 vector is a derivative of pAYC32. pAYC32 is a moderate copy- number vector and very stable, and is constructed on the basis of plasmid RSF1010 which contains a marker for streptomycin resistance (Christoserdov A. Y., Tsygankov Y. D, Broad-host range vectors derived from a RSF 1010 Tnl plasmid, Plasmid, 1986, v. 16, pp. 161-167). The pAYCTER3 vector was obtained by introduction of the polylinker from the pUC19 plasmid and the strong terminator *rrnB* into the pAYC32 plasmid instead of its promoter as follows. At first, the polylinker from pUC19 plasmid was obtained by PCR using the primers depicted in SEQ ID No. 9 and No. 10. The obtained PCR product was treated with *Eco*RI and *Bgl*II restriction enzymes. The terminator *rrnB* was also obtained by PCR using the primers depicted in SEQ ID No. 11 and No. 12. The obtained PCR product was treated with *Bgl*II and *Bcl*I restriction enzymes. Then, these two DNA fragments were ligated into pAYC32 plasmid, which had been previously treated with *Eco*RI and *Bcl*I restriction enzymes. Thus the pAYCTER3 plasmid was obtained.

### Example 3. Substitution of the native upstream region of the pckA gene with the hybrid regulatory element carrying the P_{ppsA} promoter and SD_{ϕ10} in the E. coli chromosome.

To enhance the *pckA* gene expression, the promoter region of the *ppsA* gene (P_{ppsA}) derived from the *E. coli* chromosome was linked to the Shine-Dalgarno sequence (SD sequence) of the ϕ10 gene from phage T7. This fragment was integrated upstream of the *pckA* coding region in the chromosome of the *E. coli* strain BW25113(pKD46) in place of the native region using a method described by Datsenko K.A. and Wanner B.L. (Proc.Natl.Acad.Sci.USA, 2000, 97, 6640-6645), which is also called "Red-mediated integration". In addition, the artificial DNA fragment integrated into the corresponding region of the bacterial chromosome carried chloramphenicol resistance gene (Cm^{R}) (Fig. 1). The nucleotide sequence of the substituted native region located upstream of *pckA* gene is presented in the Sequence listing (SEQ ID NO: *1). Escherichia coli* strain BW25113 containing the recombination plasmid pKD46 can be obtained from the E. coli Genetic Stock Centre, Yale University, New Haven, USA, the accession number of which is CGSC7630 (WO02103010A1).

Construction of the above-mentioned artificial DNA fragment was accomplished by the following steps. First, a DNA fragment was obtained by PCR having the *Bgl*II - restriction site in the "upstream" region, the P_{ppsA} promoter, and the SD sequence from the phage T7 ϕ10 gene linked directly to the ATG-initiation codon of the *pckA* gene in the "downstream" region. The chromosomal DNA from *E. coli* W3350 strain was as the template in PCR. PCR was conducted using primers P1 (SEQ ID NO: 13) and P2 (SEQ ID NO: 14). Primer P1 contains a *Bgl*II - restriction site. Primer P2 contains the SD sequence of the phage T7 ϕ10 gene as well as 36 nucleotides from the *pckA* open reading frame. The sequence from the *pckA* gene was introduced into primer P2 for further Red-mediated integration of the fragment into the bacterial chromosome.

In all cases, PCR was conducted using the amplificatory "TermoHybaid PCR Express PCR System". The reaction mixture having a total volume of 50 µl contains 5 µl of 10x PCR-buffer with 15 mM MgCl₂ ("Fermentas", Lithuania), 200 µM each of dNTP, 20 nM each of the exploited primers and 1 u Taq-polymerase ("Fermentas", Lithuania). 0.5 µg of the chromosomal DNA was added to the reaction mixture as the template DNA for the further PCR-driven amplification. The PCR conditions were as follows: initial DNA denaturation for 5 min at 95 °C, followed by 25 cycles of denaturation at +95 °C for 30 sec, annealing at +55 °C for 30 sec, elongation at +72 °C for 30 sec, and the final polymerization for 7 min at +72 °C.

Concurrently, the second stage of construction of the DNA fragment of interest was conducted. The Cm^{R} gene was amplified by PCR using the commercially available plasmid pACYC184 (GenBank/EMBL accession number X06403, "Fermentas", Lithuania) as the template, and primers P3 (SEQ ID NO: 15) and P4 (SEQ ID NO: 16). Primer P3 contains the BglII-restriction site used for joining the previously obtained DNA fragment containing the P_{ppsA} promoter. Primer P4 contains 36 nucleotides which are located upstream of *pckA* gene derived from *E. coli* and are necessary for further Red-mediated integration of the fragment into the bacterial chromosome.

Amplified DNA fragments were then concentrated by agarose gel-electrophoresis and extracted from the gel by centrifugation through "GenElute Spin Columns" ("Sigma", USA), and followed by ethanol precipitation.

Two obtained DNA fragments were treated with *BglII* restriction endonuclease followed by ligation using T4 DNA ligase (Maniatis T., Fritsch E.F., Sambrook, J.: Molecular Cloning: A

Laboratory Manual. 2^{nd} edn. Cold Spring Harbor, NY: Cold Spring Harbor Laboratory Press,1989).

The ligated product was amplified by PCR using primers P2 and P4. PCR was conducted in a reaction mixture having a total volume of 50 µl and containing 5 µl of 10x AccuTaq LA buffer ("Sigma",USA), 200 µM each of dNTP, 20 nM each of the exploited primers and 1 µ AccuTaq LA polymerase ("Sigma", USA). Approximately 50 ng of the ligated DNA products was added to the reaction mixture as the template DNA. The PCR conditions were as follows: initial DNA denaturation for 5 min at 95 °C followed by 25 cycles of denaturation at 95 °C for 30 sec, annealing at 55 °C for 30 sec, elongation at 72 °C for 4 min and the final polymerization for 7 min at 72 ºC.

The structure of constructed DNA region upstream of the *pckA* gene is shown in Fig.1. The nucleotide sequence of the constructed DNA region is presented in SEQ ID NO: 18.

The DNA fragment obtained and purified as described above was used for electroporation and Red-mediated integration into the bacterial chromosome of the *E. coli* strain BW25113(pKD46). The recombinant plasmid pKD46 (Datsenko, K.A., Wanner, B.L., Proc.Natl.Acad.Sci.USA, 2000, 97, 6640-6645) having the thermosensitive replicon was used as the donor of the phage λ-derived genes responsible for the Red-mediated recombination system.

The cells of BW25113(pKD46) were grown overnight at 30 °C in a liquid LB-medium also containing ampicillin (100 µg/ml), then diluted 1:100 with the SOB-medium (Yeast extract, 5 g/l; NaCl, 0.5 g/l; Tryptone, 20 g/l; KCl, 2.5 mM; MgCl₂, 10 mM) containing ampicillin (100 µg/ml) and L-arabinose (10 mM) (arabinose is used for inducing the plasmid encoding genes of Red system) and grown at 30 °C until reaching the optical density OD₆₀₀=0.4-0.7 of the bacterial culture. The grown cells from 10 ml of the bacterial culture were washed 3 times by ice-cold de-ionized water followed by suspension in 100 µl of water. 10 µl of DNA fragment (100 ng) dissolved in de-ionized water was added to the cell suspension. The electroporation was performed by "Bio-Rad" electroporator (USA) (No. 165-2098, version 2-89) according to the manufacturer's instructions. Shocked cells were added to 1-ml of SOC medium (Sambrook et al., "Molecular Cloning A Laboratory Manual, Second Edition", Cold Spring Harbor Laboratory Press (1989)), incubated 2 hours at 37 °C, and then spread onto L-agar containing 25 µg/ml of chloramphenicol. Colonies which grew within 24 h were tested for the presence of Cm^{R} marker upstream of the *pckA* gene by PCR using primers P4 (SEQ ID NO: 16) and P5 (SEQ ID NO: 17). The same colonies were also tested for the presence of the P_{ppsA} promoter region upstream of *pckA* gene by PCR using primers P1 (SEQ ID NO: 13) and P5 (SEQ ID NO: 17). For these tests, a freshly isolated colony was suspended in 20µl water, of which 1µl was used in PCR. PCR conditions were as follows: initial DNA denaturation for 10 min at 95 °C; then 30 cycles of denaturation at 95 °C for 30 sec, annealing at 55 °C for 30 sec and elongation at 72 °C for 1 min; the final polymerization for 7 min at 72 °C. A few of the Cm^{R} colonies tested contained the desired 1749 nt and 697 nt DNA fragments, which confirmed the presence of the entire constructed DNA region upstream of the *pckA* gene and the hybrid regulatory element, carrying the P_{ppsA} promoter and SD_{ϕ10} in *E. coli* chromosome, respectively. One of the obtained strains was cured from the thermosensitive plasmid pKD46 by culturing at 37 °C. The resulting strain was named *E*. *coli* strain BW25113 (Cm-P_{ppsA}-SD_{ϕ10}-pckA).

### Example 4: Effect of enhanced pckA gene expression on tryptophan production.

The tryptophan-producing *E. coli* strain SV164 (pMW-P_{lacUV5}-*serA5-fruR,* pYDDG2) was used as a parental strain for evaluating the effect of enhanced *pckA* gene expression on tryptophan production. The strain SV164 is described in detail in US patent 6,180,373.

To test the effect of enhancement of *pckA* gene expression under control of the P_{ppsA} promoter and SD of phage T7 ϕ10 gene on tryptophan production, the above-mentioned DNA fragment from the chromosome of *E. coli* strain BW25113 (Cm-P_{ppsA}-SD_{ϕ10}-*pckA*) was transferred to the tryptophan-producing *E. coli* strain SV164 (pMW*-P*_{*l*acUV5}-*serA5*-*fruR*) by P1 transduction (Miller, J.H. (1972) Experiments in Molecular Genetics, Cold Spring Harbor Lab. Press, Plainview, NY). Then, plasmid pYDDG2 was introduced into both the SV164 (pMW-P_{*lac*UV5}-*serA5-fruR*) strain and the resulting transductant.

Both the SV164 (pMW-P_{lacUV5}-*serA5-fruR,* pYDDG2) and the SV164 P_{ppsA}-SD_{ϕ10}-pckA (pMW-P_{lacUV5}-*serA5*-*fruR*, pYDDG2) strains were cultivated overnight with shaking at 37°C in 3 ml of nutrient broth supplemented with 30 µg/ml of ampicillin and 25 µg/ml of streptomycin. 0.3 ml of the obtained cultures were inoculated into 3 ml of a fermentation medium containing tetracycline (20 µg/ml) in 20 x 200 mm test tubes, and cultivated at 37°C for 48 hours on a rotary shaker at 250 rpm.

The composition of the fermentation medium is presented in Table 1.

**Table 1**

| Sections | Component | Final concentration, g/l |
|---|---|---|
| A | KH₂PO₄ | 1.5 |
| | NaCl | 0.5 |
| | (NH₄)₂SO₄ | 1.5 |
| | L-Methionine | 0.05 |
| | L-Phenylalanine | 0.1 |
| | L-Tyrosine | 0.1 |
| | Mameno (total N) | 0,07 |
| B | Glucose | 40.0 |
| | MgSO₄ • 7H₂O | 0.3 |
| C | CaCl₂ | 0.011 |
| D | FeSO₄ • 7H₂O | 0.075 |
| | Sodium citrate | 1.0 |
| E | Na₂MoO₄ • 2H₂O | 0.00015 |
| | H₃BO₃ | 0.0025 |
| | CoCl₂ • 6H₂O | 0.00007 |
| | CuSO₄ • 5H₂O | 0.00025 |
| | MnCl₂ • 4H_{z}O | 0.0016 |
| | ZnSO₄ •7 H₂O | 0.0003 |
| F | Thiamine HCl | 0.005 |
| G | CaCO₃ | 30.0 |
| H | Pyridoxine | 0.03 |

| | | |
|---|---|---|
| Section A had pH 7.1 adjusted by NH₄OH. Each section was sterilized separately. | | |

After the cultivation, the amount of L-tryptophan which accumulated in the medium was determined by TLC. 10 x 15 cm TLC plates coated with 0.11 mm layers of Sorbfil silica gel without fluorescent indicator (Stock Company Sorbpolymer, Krasnodar, Russia) were used. Sorbfil plates were developed with a mobile phase: propan-2-ol : ethylacetate : 25% aqueous ammonia : water = 16 : 16 : 3 : 9 (v/v). A 2% solution of ninhydrin in acetone was used as a visualizing reagent.

The results are presented in the Table 2.

**Table 2**

| *E. coli* strain | OD₆₀₀ | Amount of tryptophan, g/l |
|---|---|---|
| SV164 (pMW-P_{*lac*UV5}*-serA5-fruR,* pYDDG2) | 7.5 | 4.20 |
| SV164 P_{ppsA}-SD_{ϕ10}-*pckA* (pMW-P_{*lac*UV5}*-serA5-fruR,* pYDDG2) | 7.5 | 4.61 |

As it can be seen from the Table 2, the enhancement of *pckA* gene expression improved tryptophan productivity of the SV164 (pMW-P_{*lac*UV5}*-serA5-fruR*) strain.

### Industrial Applicability

According to the present invention, productivity of an aromatic L-amino acid-producing strain can be enhanced and a method for producing the aromatic L-amino acid using the strain is provided. The present invention is useful in the fermentation industry.

## Claims

1. A method for producing an aromatic L-amino acid selected from the group consisting of L-tryptophan, L-phenylalanine and L-tyrosine by fermentation comprising cultivating an L-amino acid-producing bacterium of *Escherichia coli,* wherein the bacterium has been modified to have enhanced activity of phosphoenol pyruvate (PEP) carboxykinase, in a culture medium to cause accumulation of L-amino acid in the culture medium, and collecting the L-amino acid from the culture medium, wherein the activity of PEP carboxykinase is enhanced by modifying an expression control sequence of the PEP carboxykinase gene on the chromosome of the bacterium so that the expression of the gene is enhanced, or by increasing copy number of the gene, and wherein the bacterium is further modified to have enhanced expression of *yddG* open reading frame by increasing the copy number of the *yddG* open reading frame, wherein the *yddG* gene can be cloned using the primers depicted in SEQ ID No. 7 and No. 8.

2. The method according to claim 1, wherein a native promoter of said PEP carboxykinase gene is replaced with more potent promoter.

3. The method according to claim 2, wherein said more potent promoter is ppsA promoter which is activated by FruR protein.

4. The method according to claim 1, wherein a native Shine-Dalgarno (SD) sequence of said PEP carboxykinase gene is replaced with a more efficient SD sequence.

5. The method according to claim 4, wherein said more potent SD sequence is SD sequence of ϕ10 gene from phage T7.

6. The method according to any one of claims 1 to 5, wherein the PEP carboxykinase gene encodes the following protein (A) or (B):
(A) a protein, which comprises the amino acid sequence shown in SEQ ID NO: 2 in Sequence listing;
(B) a protein which comprises the amino acid sequence shown in SEQ ID NO: 2 in Sequence listing including deletion, substitution, insertion or addition of one to ten amino acids, and which has an activity of PEP carboxykinase.

7. The method according to any one of claims 1 to 5, wherein the PEP carboxykinase gene comprises the following DNA (a) or (b):
(a) a DNA which comprises the nucleotide sequence of the nucleotides 1 to 1623 in SEQ ID NO: 1; or
(b) a DNA which is hybridizable with the nucleotide sequence of the nucleotides 1-1623 in SEQ ID NO:1 or a probe which can be prepared from said nucleotide sequence under stringent conditions and encodes a protein having an activity of PEP carboxykinase, wherein the stringent conditions are conditions in which washing is performed at 60°C, and at a salt concentration corresponding to 1 x SSC and 0.1% SDS.

8. The method according to any one of claims 1 to 5, wherein the PEP carboxykinase gene encodes a protein which comprises the amino acid sequence shown in SEQ ID NO: 2 in Sequence Listing.

9. The method according to any one of claims 1 to 5, wherein the PEP carboxykinase gene comprises the nucleotide sequence of the nucleotides 1 to 1623 in SEQ ID NO: 1.

10. The method according to any one of claims 1 to 9, wherein the bacterium has enhanced expression of other genes for aromatic amino acid biosynthesis.

## Patentansprüche

1. Verfahren zum Herstellen einer aromatischen L-Aminosäure, die aus der Gruppe ausgewählt ist, die aus L-Tryptophan, L-Phenylalanin, und L-Tyrosin besteht, durch Fermentation, welches das Kultivieren eines L-Aminosäure produzierenden Bakteriums von Escherichia coli, wobei das Bakterium so modifiziert worden ist, dass die Aktivität von Phosphoenolpyruvat (PEP)-Carboxykinase erhöht ist, in einem Kulturmedium zur Anhäufung der L-Aminosäure in dem Kulturmedium und das Gewinnen der L-Aminosäure aus dem kulturmedium umfasst, wobei die Aktivität der PEP-Carboxykinase durch Modifizieren einer Expressionskontrollsequenz des PEP-Carboykinase-Gens auf dem Chromosom des Bakteriums, sodass die Expression des Gens erhöht ist, oder durch Erhöhen der Kopienzahl des Gens verstärkt ist, und wobei das Bakterium außerdem so modifiziert worden ist, dass die Expression des offenen Leserahmens von yddG durch Erhöhen der Kopienzahl des offenen Leserahmens von yddG verstärkt ist, wobei das yddG-Gen unter Einsatz der in SEQ-ID-Nr. 7 und 8 angegebenen Primer kloniert werden kann.

2. Verfahren nach Anspruch 1, wobei ein nativer Promotor des PEP-Carboxykinase-Gens durch einen stärkeren Promotor ersetzt wird.

3. Verfahren nach Anspruch 2, wobei der stärkere Promotor der ppsA-Promotor ist, der durch das FruR-Protein aktiviert wird.

4. Verfahren nach Anspruch 1, wobei eine native Shine-Dalgarno (SD)-Sequenz des PEP-Carboxykinase-Gens durch eine effizientere SD-Sequenz ersetzt wird.

5. Verfahren nach Anspruch 4, wobei die stärkere SD-Sequenz die SD-Sequenz des ϕ10-Gens des Phagen T7 ist.

6. Verfahren nach einem der Ansprüche 1 bis 5, wobei das PEP-Carboxykinase-Gen das folgende Protein (A) oder (B) kodiert:
(A) ein Protein, das die SEQ-ID-Nr. 2 der Sequenzliste gezeigte Aminosäuresequenz enthält;
(B) ein Protein, das die in SEQ-ID-Nr. 2 der Sequenzliste gezeigte Aminosäuresequenz enthält, einschließlich Deletion, Substitution, Insertion oder Addition von einer bis zehn Aminosäuren, und das eine Aktivität vom PEP-Carboxykinase hat.

7. Verfahren nach einem der Ansprüche 1 bis 5, wobei das PEP-Carboxykinase-Gen die folgende DNA (a) oder (b) umfasst:
(a) eine DNA, welche die Nukleotidsequenz der Nukleotide 1 bis 1 623 in SEQ-ID-Nr. 1 enthält; oder
(b) eine DNA, die mit der Nukleotidsequenz der Nukleotide 1 bis 1623 der SEQ-ID-Nr. 1 oder mit einer Sonde, die aus dieser Nukleotidsequenz hergestellt werden kann, unter stringenten Bedingungen hybridisierbar ist und für ein Protein kodiert, das die Aktivität von PEP-Carboxykinase aufweist, wobei die stringenten Bedingungen Bedingungen sind, in denen Waschen bei 60°C bei einer Salzkonzentration, die 1 x SSC und 0,1% SDS entspricht, durchgeführt wird.

8. Verfahren nach einem der Ansprüche 1 bis 5, wobei das PEP-Carboxykinase-Gen für ein Protein kodiert, das die in SEQ-ID-Nr. 2 des Sequenzprotokolls gezeigte Aminosäuresequenz umfasst.

9. Verfahren nach einem der Ansprüche 1 bis 5, wobei das PEP-Carboxykinase-Gen die Nukleotidsequenz der Nukleotide 1 bis 1623 der SEQ-ID-Nr. 1 enthält.

10. Verfahren nach einem der Ansprüche 1 bis 9, wobei das Bakterium eine verstärkte Expression von anderen Genen für die Biosynthese einer aromatischen Aminosäure aufweist.

## Revendications

1. Un procédé de production d'un L-acide aminé aromatique choisi parmi le groupe constitué par du L-tryptophane, de la L-phénylalanine et de la L-tyrosine par fermentation comprenant la culture d'une bactérie *Escherichia coli* produisant des L-acides aminés , dans lequel la bactérie a été modifiée pour présenter une activité améliorée de la phosphoénol pyruvate (PEP) carboxykinase, dans un milieu de culture pour entraîner l'accumulation de L-acide aminé dans le milieu de culture, et la collecte du L-acide aminé du milieu de culture, dans lequel l'activité de la PEP carboxykinase est accrue par la modification d'une séquence de contrôle de l'expression du gène de la PEP carboxykinase sur le chromosome de la bactérie de sorte que l'expression du gène soit accrue, ou par l'augmentation du nombre de copies du gène, et dans lequel la bactérie est en outre modifiée de manière à présenter une expression accrue du cadre ouvert de lecture de *yddG* en augmentant le nombre de copies du cadre ouvert de lecture de *yddG,* dans lequel le gène *yddG* peut être cloné à l'aide des amorces illustrées dans les SEQ ID N0 7 et N0 8.

2. Le procédé selon la revendication 1, dans lequel un promoteur natif dudit gène de la PEP carboxykinase est remplacé par un promoteur plus puissant.

3. Le procédé selon la revendication 2, dans lequel ledit promoteur plus puissant est le promoteur de ppsA qui est activé par la protéine FruR.

4. Le procédé selon la revendication 1, dans lequel une séquence de Shine-Dalgarno (SD) native dudit gène de la PEP carboxykinase est remplacée par une séquence SD plus efficace.

5. Le procédé selon la revendication 4, dans lequel ladite séquence SD plus puissante est la séquence SD du gène ϕ10 du phage T7.

6. Le procédé selon l'une quelconque des revendications 1 à 5, dans lequel le gène de la PEP carboxykinase code pour la protéine (A) ou (B) suivante :
(A) une protéine qui comprend la séquence d'acides aminés illustrée dans la SEQ ID NO : 2 de la liste des séquences ;
(B) une protéine qui comprend la séquence d'acides aminés illustrée dans la SEQ ID NO : 2 de la liste des séquences incluant la délétion, la substitution, l'insertion ou l'addition de un à dix acides aminés, et qui a une activité de la PEP carboxykinase.

7. Le procédé selon l'une quelconque des revendications 1 à 5, dans lequel le gène de la PEP carboxykinase comprend l'ADN (a) ou (b) suivant :
(a) un ADN qui comprend la séquence nucléotidique des nucléotides 1 à 1623 de la SEQ ID NO : 1 ; ou
(b) un ADN qui peut être hybridé avec la séquence nucléotidique des nucléotides 1 à 1623 de la SEQ ID NO : 1 ou une sonde qui peut être préparée à partir de ladite séquence nucléotidique dans des conditions strictes et qui code pour une protéine ayant une activité de la PEP carboxykinase, les conditions strictes étant des conditions dans lesquelles le lavage est réalisé à 60 °C, et à une concentration de sel correspondant à 1 x SSC et 0,1 % de SDS.

8. Le procédé selon l'une quelconque des revendications 1 à 5, dans lequel le gène de la PEP carboxykinase code pour une protéine qui comprend la séquence d'acides aminés illustrée dans la SEQ ID NO : 2 de la liste des séquences.

9. Le procédé selon l'une quelconque des revendications 1 à 5, dans lequel le gène de la PEP carboxykinase comprend la séquence nucléotidique des nucléotides 1 à 1623 de la SEQ ID NO : 1.

10. Le procédé selon l'une quelconque des revendications 1 à 9, dans lequel la bactérie présente une expression accrue des autres gènes pour la biosynthèse d'acides aminés aromatiques.
